# EUROPEAN PATENT APPLICATION

(11) **EP 1 025 816 A1**
(43) Date of publication of application: **09.08.2000**
(21) Application number: 00300782.0
(22) Date of filing: 01.02.2000
(51) Int. Cl.: A61F 2/36

(54) **Femoral component for use in a replacement hip joint**

(30) Priority: 02.02.1999 GB 9902321
(71) Applicant: BENOIST GIRARD ET CIE, 14201 Hérouville-Saint-Clair Cedex (FR)
(72) Inventor: Storer, John Andrew, 14250 Jouyae Mondaye, Bayeux (FR); Gie, Graham Allan, Yeoford, Devon EX17 5EZ (GB); Ling, Robin Sydney Mackwood, Prof., Dartmouth, Devon TQ6 0HB (GB); Timperley, Andrew John, ST. Leonard's, Exeter EX2 4PA (GB)
(74) Representative: Bridge-Butler, Alan James

(57) **Abstract**

A femoral component (1) of a replacement hip joint which has a tapered collarless stem (2) for fixing in a medullary canal by cement and in which the anterior (6) and posterior (7) surfaces of said stem are respectively substantially concave and convex over at least a part of their respective proximal to distal lengths.

## Description

This invention relates to a femoral component for use in a replacement hip joint.

The Exeter type femoral component of the kind shown in British Patent Specification No. 1 409 054 is well known and comprises a neck which carries a ball head or head spigot for co-operation with an acetabular prosthesis. The neck is connected to a tapered collarless stem.

The Exeter type of femoral component is used with bone cement and is typically inserted into a pre-prepared medullary canal filled with bone cement. Despite rasping the medullary canal with a rasp which is the same shape as the stem but slightly larger, the natural shape of the femur may conspire to leave a deficient cement mantle anteriorly after the prosthesis has been inserted. A deficient mantle is undesirable as studies have shown that it may be associated with localised osteolysis. A curved stem might allow for a more even cement mantle thus decreasing the risk of a deficient mantle.

It is proposed that by manufacturing the prosthetic component with a curve in the anterior direction the risk of incorrect insertion will be reduced.

According to the present invention a femoral component of a replacement hip joint which has a tapered collarless stem for fixing in a medullary canal by cement and in which the anterior and posterior surfaces thereof are respectively substantially concave and convex over at least part of their respective proximal to distal lengths.

If desired the anterior and posterior surfaces can be respectively substantially concave and convex over at least the major portion of their respective proximal to distal lengths.

Thus the overall curve of the component allows it to be inserted into the medullary canal without impinging against the walls and allows a desirable mantle of cement around the component once in position.

The anterior and posterior surfaces can be respectively substantially concave and convex over the entire proximal to distal length of the stem.

Alternatively the anterior and posterior surfaces can be respectively substantially concave and convex over the entire proximal to distal length of the component, thus including the neck.

Preferably the respectively concave and convex curved anterior and posterior surfaces describe arcs of constant radius about a line substantially perpendicular to an anterior/posterior plane which passes through the general axis of the component.

With this arrangement the respectively concave and convex curved anterior and posterior surfaces can be arranged to describe arcs of constant radii about different lines substantially perpendicular to the anterior posterior plane.

In another convenient construction the respectively concave and convex curved anterior and posterior surfaces can describe arcs of blended differing radii about differing lines situated perpendicular to the said anterior/posterior plane.

The anterior/posterior plane can be substantially perpendicular to the medial/lateral plane of the component and parallel with the proximal/distal plane thereof.

In an alternative construction the femoral component of a replacement hip joint which has a tapered collarless stem for fixing in a medullary canal by cement can have anterior and posterior surfaces which are respectively substantially concave and convex over at least part of their respective proximal to distal lengths and in which at least part of the remaining portion of the anterior and posterior surfaces are respectively convex and concave.

Thus the component may be curved first in one direction and then in the opposite direction resulting in an extended 'S' shape.

The invention can be performed in various ways and two embodiments will now be described by way of example and with reference to the accompanying drawings in which :-
Figure 1 is a representation of a right side prosthetic femoral component according to the invention showing the surface of the medial side;
Figure 2 is a representation of an alternative embodiment of a right side prosthetic component according to the invention also showing the surface of the medial side;
Figure 3 is a representation of a right side prosthetic component as in either of Figures 1 & 2 showing the posterior side; and,
Figure 4 is a representation of an alternative construction of a right side prosthetic component according to the invention also showing the surface of the medial side.

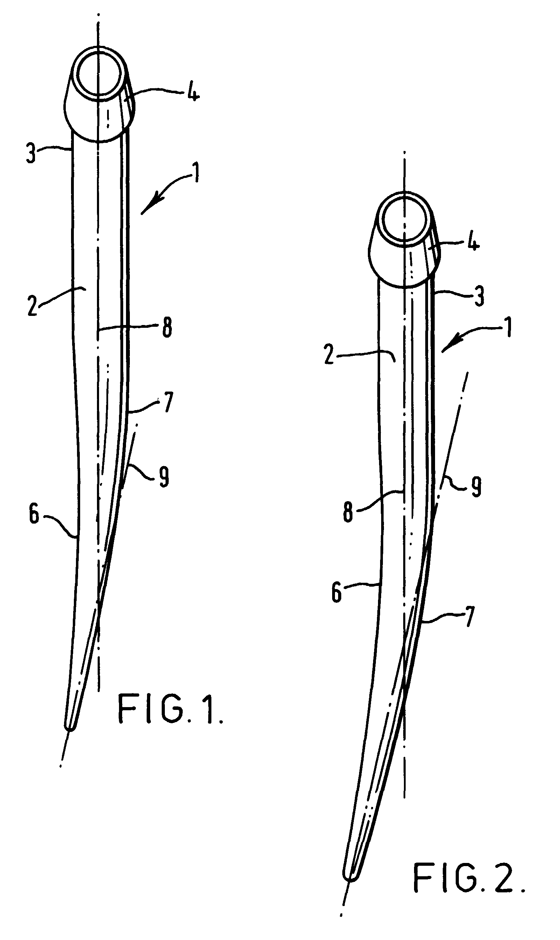

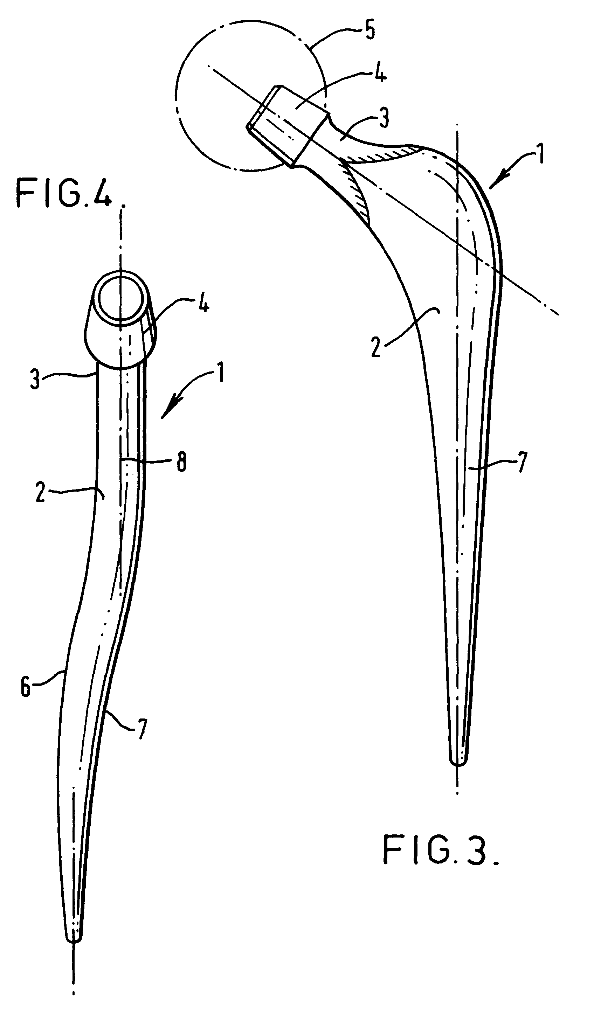

As shown in Figures 1 to 4 a femoral component 1 of a replacement hip joint has a tapered collarless stem 2 for fixing in a medullary canal by cement, and a neck 3 carrying a spigot 4 to receive a ball head 5 (not shown in Figures 1 and 2). The anterior 6 and posterior 7 surfaces of the stem 2 are respectively substantially concave and convex over at least part of their respective proximal to distal lengths.

The component 1 shown in Figure 2 can clearly be seen to have a smaller radius of curvature over the anterior 6 and posterior 7 surfaces than the component 1 shown in figure 1 to indicate how the shape of the component can be varied and supplied in different configurations according to the requirements of the patient.

The components are "handed" according to whether they are for use in either a left or right femur.

In figures 1 to 4 the component is shown with only the stem 2 of the component being curved according to the invention, but it is also within the scope of the invention for the neck 3 to be curved in such a way as to be a continuation of the curve of the stem, so that the entire proximal/distal length of the component is curved.

As shown in Figures 1 and 2 the general proximal/distal longitudinal axis of the component is indicated by chain line 8 and the general curving offset is indicated by chain line 9. It will be appreciated that chain line 9 is shown as a straight line rather than curved to emphasize the degree of offset.

In the construction shown in Figure 1 the offset curved portion extends over about one third of the total length of the component and in Figure 2 the curvature extends over about half the length of the stem. In each case the respectively concave and convex curved anterior and posterior surfaces 6 and 7 describe arcs of constant radius about a line substantially perpendicular to an anterior/posterior plane which passes through the general axis 8 of the component.

Alternatively these concave and convex anterior and posterior surfaces 6 and 7 could describe arcs of constant radii about different lines substantially perpendicular to the said anterior/posterior plane.

Again, if desired, these curved surfaces could describe arcs of blended differing radii about different lines situated perpendicular to the said anterior/posterior plane.

It will be seen that the anterior/posterior plane referred to is substantially perpendicular to the medial/lateral plane of the component and parallel with the proximal/distal plane thereof.

Although the component is shown with a spigot to receive a ball head 5, the ball head 5 could be integral with the neck 3.

In the construction shown in the drawings the concave 6 and convex surfaces 7 are symmetrically and equally spaced about a curved proximal/distal axis but if desired and for varying clinical reasons it might be desirable for them to be spaced away from such a curved axis by different dimensions to produce an asymmetric construction. This can apply whether the curved axis is about a single radius or a radius of varying dimensions or about radii which emanate from different points.

As seen in figure 4 a different construction of the invention is shown in which the anterior 6 surface is curved both concavely and convexly and the posterior 7 surfaces is curved both convexly and concavely, thus giving the component 1 a substantially extended 'S' shape. The curved surfaces describe arcs which can be defined in a similar manner as described above with respect to said anterior/posterior plane.

## Claims

1. A femoral component of a replacement hip joint which has a tapered collarless stem for fixing in a medullary canal by cement and in which the anterior and posterior surfaces of said stem are respectively substantially concave and convex over at least a part of their respective proximal to distal lengths.

2. A femoral component as claimed in claim 1 in which said anterior and posterior surfaces of said stem are substantially concave and convex over at least a major portion of their respective proximal to distal lengths.

3. A femoral component as claimed in claim 1 in which the anterior and posterior surfaces of said stem are respectively substantially concave and convex over the entire proximal to distal length of the stem.

4. A femoral component as claimed in claim 1 in which the anterior and posterior surfaces thereof are respectively substantially concave and convex over the entire proximal to distal length of the component.

5. A femoral component as claimed in claim 1 in which the anterior and posterior surfaces of said stem are respectively substantially concave and convex over at least part of their respective proximal to distal lengths and at least part of the remaining portion of said anterior and posterior surfaces are respectively convex and concave.

6. A femoral component as claimed in any one of the preceding claims 1 to 5 in which the respectively concave and convex curved anterior and posterior surfaces describe arcs of constant radius about a line substantially perpendicular to an anterior/posterior plane which passes through the general axis of the component.

7. A femoral component as claimed in claim 6 in which the respectively concave and convex curved anterior and posterior surfaces describe arcs of constant radii about different lines substantially perpendicular to said anterior/posterior plane.

8. A femoral component as claimed in claim 6 in which the respectively concave and convex curved anterior and posterior surfaces describe arcs of blended differing radii about differing lines situated perpendicularly to said anterior/posterior plane.

9. A femoral component as claimed in claim 6, claim 7 or claim 8 in which the anterior/posterior plane is substantially perpendicular to the medial/lateral plane of the component and parallel with the proximal/distal plane thereof.

10. A femoral component as claimed in any one of the preceding claims 1 to 9 in which said concave and convex anterior and posterior surfaces are symmetrically spaced about a curved proximal/distal axis.

11. A femoral component as claimed in any one of preceding claims 1 to 9 in which said concave and convex anterior and posterior surfaces are spaced about a curved proximal/distal axis by different dimensions to produce an asymmetric construction.
